# EUROPEAN PATENT SPECIFICATION

(11) **EP 3 431 010 B1**
(45) Date of publication and mention of the grant of the patent: **24.06.2020**
(21) Application number: 18175805.3
(22) Date of filing: 10.03.2015
(51) Int. Cl.: A61B 10/00, A63H 27/10, A61B 5/155, A61B 5/15, B65B 3/00

(54) **APPARATUS, SYSTEM AND METHOD FOR FILLING CONTAINERS WITH FLUIDS**
VORRICHTUNG, SYSTEM UND VERFAHREN ZUM BEFÜLLEN VON BEHÄLTERN MIT FLÜSSIGKEITEN
APPAREIL, SYSTÈME ET PROCÉDÉ DE REMPLISSAGE DE RÉCIPIENTS AVEC DES FLUIDES

(30) Priority: 07.02.2014 US 201461937083 P; 20.02.2014 US 201461942193 P; 22.09.2014 US 201414492487
(43) Date of publication of application: 23.01.2019
(62) Divisional of application: 15158482.8
(73) Proprietor: Tinnus Enterprises, LLC, Plano, TX 75074 (US)
(72) Inventor: MALONE, Joshua, Plano, Texas 75074 (US)
(74) Representative: Dentons UK and Middle East LLP

(56) References cited:
- EP-A2- 1 548 420
- FR-A1- 2 911 512
- US-A- 5 234 726
- US-A1- 2011 253 256
- US-A1- 2013 118 640
- US-A1- 2013 226 219
- Anonymous: "Bunch O Balloons: 100 Water Balloons in Less Than 1 Minute by Tinnus Enterprises - Kickstarter", , 26 July 2014 (2014-07-26), XP055407607, Retrieved from the Internet: URL:https://web.archive.org/web/2014072604 0504/https://www.kickstarter.com/projects/ bunchoballoons/bunch-o-balloons-100-water- balloons-in-less-than-1 [retrieved on 2017-09-18]

## Description

### TECHNICAL FIELD

The present disclosure relates generally to fluid inflatable systems and more particularly, to an apparatus, system and method for filling containers with fluids.

### BACKGROUND

Inflatable containers such as balloons can be filled with a variety of fluids, such as air, helium, water, medicines, etc. In some cases, it may be desirable for a lot of inflatable containers to be filled with fluids. For example, festive balloons used as props in conventions, large parties, etc. may number in the hundreds and may require substantial human effort to fill them all in a timely manner. In another example, water balloons used as kids' toys may need to be filled in large numbers to aid in various games. Various methods may be employed to fill such inflatable containers. For example, an individual may sequentially blow up and tie each balloon by hand or use a tank of compressed air or helium to inflate the balloon, which then has to be tied. This sequential filling is time consuming. In another example, an individual may fill water balloons with water by hand one at a time, and then tie the balloons, which can all be quite time-consuming. Moreover, the inflatable containers may be damaged or filled to different volumes.

It is therefore an object of the present invention to provide an apparatus, system and method for filling containers with fluids that addresses at least some of the above mentioned disadvantages and/or that will at least provide the public with a useful choice.

US2013118640 (A1) discloses a multi-balloon filling assembly including a water supply fitting, a main conduit, lateral conduits, a plurality of conduit tips, stop valves, and a quick-fill switch.

### BRIEF DESCRIPTION OF THE INVENTION

In one aspect the present invention provides apparatus for simultaneously filling a plurality of inflatable containers with liquids comprising: a housing having a first end at which there is an opening configured for connection to a fluid supply, and a plurality of holes from which a plurality of hollow tubes extend, each of the plurality of hollow tubes having a respective one of the plurality of inflatable containers removably attached at an end thereto to facilitate substantially simultaneous filling of the plurality of containers from the fluid supply, characterised in that:
each one of said plurality of inflatable containers is clamped about the end of its respective one of the plurality of hollow tubes by an elastic ring, the elastic rings are configured to release the inflatable containers from their respective hollow tubes when the inflatable containers have reached a desired size, by one or more of:
   i) the inflatable containers falling off the tube;
   ii) manual shaking the inflatable containers off the tube by a user; and
   iii) the inflatable containers being manually pulled away from the tube by a user,
and each elastic ring is configured to seal its respective one of the plurality of inflatable containers upon detachment of the inflatable container from its corresponding hollow tube.

The first end of the housing may have an outermost perimeter that is smaller in length than an outermost perimeter of the second end.

The opening at the first end of the housing may have a threaded inner surface.

Each container may comprise an expandable balloon portion.

Each container may comprise a rigid portion and a flexible portion, the flexible portion disposed between the elastic fastener and the end of a respective one of the plurality of tubes.

Each container may comprise a volumetric measurement marking providing a visual reference for filling the container to a desired volume.

The elastic fastener may be disposed outwardly from the container and clamp an inner surface of the container against an outer surface of the respective one of the plurality of tubes.

The fluid may comprise one or more of water, air, and helium.

Each elastic fastener may comprise an o-ring configured to automatically seal the container in response to a force applied to the container in a direction away from the housing.

The hollow tubes may be flexible.

The plurality of tubes may comprise a first set of tubes each having a first length, and a second set of tubes each having a second length longer than the first length.

The housing may be attached to a valve coupled to a fluid source, wherein the valve is configured to control delivery of the fluid to fill the plurality of containers.

The valve may include a lever that can be turned to a first position to turn on the valve and allow fluid flow to the housing, wherein the lever can be turned to a second position to turn off the valve and stop fluid flow to the housing.

One end of the valve may be connected to a hose attached to a water supply, and the other end is threaded to the housing.

In a second aspect the present invention provides a method for simultaneously filling a plurality of inflatable containers with liquids, comprising at least the steps of:
A. attaching a housing to a liquid supply via an opening at a first end of the housing, said housing further comprising a plurality of holes from which a plurality of hollow tubes extend, each of the plurality of hollow tubes having a respective one of the plurality of inflatable containers removably attached at an end thereto to facilitate substantially simultaneous filling of the plurality of containers from the fluid supply, and wherein each one of said plurality of inflatable containers is clamped about the end of its respective one of the plurality of hollow tubes by an elastic ring configured to restrict detachment of the inflatable container from the hollow tube,
B. simultaneously filling each of said plurality of inflatable containers with liquid,
C. detaching the plurality of inflatable containers from their respective tube ends when the inflatable containers have reached a desired size by one or more of:
   i) falling off the tube;
   ii) manual shaking by a user; or
   iii) being manually pulled away from the tube by a user; and
D. allowing the elastic rings to seal the plurality inflatable containers with the liquid inside after release from the hollow tubes.

There is also disclosed herein apparatus comprising a tube removably joined to at least one elastic container by an open elastic valve, wherein the tube facilitates filling the elastic container with a fluid, the elastic valve being configured to automatically close upon removal of the tube from the elastic container to seal the fluid inside the elastic container.

Preferably the elastic valve comprises an elastic ring disposed around a neck of the elastic container.

There is also disclosed herein an apparatus comprising a plurality of flexible tubes, each flexible tube joined to a balloon, wherein the flexible tubes facilitate filling the balloons with a fluid, wherein the balloons are disposed in sufficient proximity to one another to push on each other during their filling, thereby causing the tubes to flex.

Preferably the flexible tubes are of different lengths.

There is also disclosed herein a balloon filling apparatus comprising a plurality of tubes and a plurality of balloons, wherein each balloon is connected to a respective one of the tubes with a connecting force not less than a weight equivalent to one of the balloons if substantially filled with water, the connecting force being overcomeable by applying an upward acceleration on the tube, wherein the apparatus is configured to fill the plurality of the balloons substantially simultaneously.

Preferably the connecting force is less than 1 Newton.

Preferably the connecting force is provided by an elastic valve configured to automatically seal the balloon if the connecting force is overcome.

Preferably each hole of the plurality of holes at the second end of the housing extends through an outer surface of the housing, the outer surface opposing the opening at the first end of the housing.

There is also disclosed herein an apparatus comprising or including
a conduit or manifold with at least one outlet and with an inlet for fluid to be ducted to egress from the or each outlet, and
a resiliently collared, resiliently banded and/or resiliently beaded fluid inflatable container constrained by the collar, band and/or bead such that its fluid entranceway is releasably about, but sealed to, the or its outlet;
the arrangement being that fluid can pass through such conduit or manifold in use to inflate the container(s) whilst the collar, band and/or bead of the or each container keeps the fluid entranceway about and sealed to the or its outlet;
and the arrangement further being such that the fluid content weight and/or inertia can assist or cause the release by axial separation of the fluid entranceway of the or each container from the or its outlet whereupon the or each collar, band and/or bead, no longer expanded by the or its tubular outlet, closes the or its fluid entranceway.

Preferably the outlet is tubular.

There is also disclosed herein apparatus comprising
a manifold or manifold assembly ('manifold') providing an ability to duct fluid ingressing via an inlet to egress out of each of a plurality of outlets,
a plurality of fluid inflatable containers each having its entranceway about a said outlet, and
a resilient collar, band and/or bead of and/or about each entranceway in a stretched condition thereby to hold the entranceway sufficiently tightly about its outlet to receive a fluid fill but not so tightly that the fluid fill weight and/or inertia cannot assist and/or cause the release and/or extraction of its outlet and thereafter the fluid tight sealing of the tubular entranceway by a contraction of its collar, band and/or bead.

Preferably the inlet is a threaded inlet.

Preferably the outlets are each tubular.

Preferably the entrance way is tubular.

There is also disclosed herein a water balloon assembly comprising
a plurality of balloons each with a tubular entranceway, and
a collar, band or bead about or of each tubular entranceway, and
a manifold or manifold assembly ('manifold') having an inlet to receive water and duct such water out of each of a plurality of tubular outlets;
wherein each balloon is held sufficiently tightly by the collar, band or bead with the liquid entranceway about its tubular outlet of the manifold that water will pass out of its tubular outlet through its tubular entranceway to inflate the balloon;
and wherein each balloon can have its tubular entranceway sealed by a contraction of its collar, band or bead when no longer held to it's tubular outlet;
and wherein separation of a water inflated balloon from its manifold outlet can be caused or assisted by the weight or inertia of the water in the balloon.

There is also disclosed herein an apparatus comprising or including
a plurality of resiliently chokered water inflatable balloons each held by its choker about its tubular entranceway, and as a sleeve, on an outlet tube of a filling manifold connectable at its inlet to a water source.

Preferably the choker hold on each manifold outlet enables post water inflation axial separation of the sleeved tubular entranceway from its tubular outlet and subsequent choker sealing of the tubular entranceway of the water inflated balloon.

Preferably the weight and/or inertia of the water content assists and/or causes the axial separation.

There is also disclosed herein a filling procedure for multiple balloons, each to be sealed by a constrictor about its tubular entranceway, the procedure comprising the steps of
providing a manifold with an inlet to receive water and multiple tubular outlets, each outlet having a tubular entranceway, as a sleeve, held by the constrictor on the tubular entranceway, and
inflating each balloon with water and allowing drop off and/or causing drop off of each balloon to self seal reliant on its constrictor.

There is also disclosed herein a water filled balloon sealed by a constrictor about its tubular entranceway.

There is also disclosed herein a plurality of juxtaposed water filled balloons each in fluid communication via a manifold to a single water supply conduit.

There is also disclosed herein a water filled balloon sealed by a constrictor about its tubular entranceway, such constrictor having effected the seal after a sliding off of the tubular entranceway from a filling spigot.

Preferably the filling spigot was a tubular outlet of a manifold thread engageable to a suitable tap.

There is also disclosed herein a water filled balloon sealed by a constrictor about its beaded tubular entranceway, the beading assisting constrictor location about the entranceway.

There is also disclosed herein an item of commerce, comprising
(1) apparatus as herein before described and
(2) packaging that has the apparatus therein so as not to allow easy separation of each balloon or container from its associated tubular outlet from the manifold.

Preferably the apparatus allows a thread engagement of the inlet of the manifold to complementary faucet without separation of any balloon or container from the manifold.

There is also disclosed herein apparatus suitable to provide a multiple of water filled and choker or constrictor sealed balloons upon water filling of the balloons using a manifold of the apparatus, the apparatus comprising a said manifold with a inlet to receive water and multiple tubular outlets out which water is to issue, a balloon docked about each tubular outlet to receive issuing water, and a said choker or constrictor of each balloon to serve the multiple functions:
(a) hold its balloon to receive issuing water,
(b) allow dedocking of its balloon,and
(c) choke or constrictor seal water content in its balloon as the water filled balloon is being dedocked and/or upon dedocking.

Preferably the hollow tubes as herein before described are rigidly flexible.

Preferably the tubes are able to bend, yet keep their non bent cross sectional shape.

Preferably the balloons, in their pre-filled condition, allow the distal ends of the tubes at where the balloons are engaged, to be in more condensed configuration compared to when the balloons filled.

Preferably the tubes extend substantially parallel each other and are adapted and configured, when being filled, to progressively splay outwards.

Preferably the tubes extend from the housing generally in the same direction and are adapted and configured, when being filled, to progressively splay outwards.

Preferably the tubes are adapted to not crease, fold or buckle when the balloons are being filled.

Preferably the outward splay is as a result of the expansion of the girth of the balloons as they are filled and the balloons being in contact with adjacent of said balloons.

Preferably the balloons do not contain any liquid prior to being filled with water.

Preferably the balloons contain air prior to being filled with water.

Preferably the air in the balloons is in fluid connection with ambient air.

Preferably there is no pressure differential between the air in the balloons and ambient air.

Preferably balloons, prior to being filled with water, are flaccid.

Preferably the balloons, once filled, are volumetrically, at least 3 times their flaccid size.

Preferably the balloons, once filled, are volumetrically, at least 5 times their flaccid size.

Preferably the balloons, once fully filled, are no more than 0.002 cubic metres.

Preferably the balloons, once fully filled, are no more than 0.0014 cubic metres.

Preferably the balloons, once fully filled, are no more than 0.0005 cubic metres.

Preferably the balloons, once fully filled, are no more than 0.0001 cubic metres.

Preferably the balloons comprise of a flexible body and a less flexible spout, the spout being engaged to a respective tube.

Preferably the flexible body or each balloon is located beyond the distal end of a respective tube.

Preferably a first group of tubes have their distal ends more proximate the housing/manifold than a second group of tubes, thereby locating a first group of balloons more proximate the housing/manifold than a second group of balloons.

Preferably the first group of tubes is located about the second group of tubes.

Preferably the tubes are arranged, at the housing/manifold in arrays of concentric circles.

Preferably the second group of tubes are of inner more concentric circles to the first group of tubes.

Preferably the ends of the tubes displace, upon filling of the balloons, to be more than 20mm apart each other.

Preferably the ends of the tubes displace, upon filling of the balloons, to be more than 25mm apart each other.

Preferably the ends of the tubes displace, upon filling of the balloons, to be more than 30mm apart each other.

Preferably the ends of the tubes displace, upon filling of the balloons, to be more than 35mm apart each other.

Preferably the ends of the tubes displace, upon filling of the balloons, to be more than 40mm apart each other.

Preferably the ends of the tubes displace, upon filling of the balloons, to be more than 45mm apart each other.

Preferably the ends of the tubes displace, upon filling of the balloons, to be more than 50mm apart each other.

Preferably the ends of the tubes displace, upon filling of the balloons, to be more than 55mm apart each other.

Preferably the ends of the tubes displace, upon filling of the balloons, to be more than 60mm apart each other.

Preferably the ends of the tubes displace, upon filling of the balloons, to be more than 65mm apart each other.

Preferably the ends of the tubes displace, upon filling of the balloons, to be no more than 80mm apart each other.

Preferably the ends of the tubes displace, upon filling of the balloons, to be no more than 70mm apart each other.

Preferably the ends of the tubes displace, upon filling of the balloons, to be no more than 65mm apart each other.

Preferably the ends of the tubes, when the balloons are flaccid, have a spacing of less than 50mm.

Preferably the ends of the tubes, when the balloons are flaccid, have a spacing of less than 40mm.

Preferably the ends of the tubes, when the balloons are flaccid, have a spacing of less than 30mm.

Preferably the ends of the tubes, when the balloons are flaccid, have a spacing of less than 20mm.

### BRIEF DESCRIPTION OF THE DRAWINGS

To provide a more complete understanding of the present disclosure and features and advantages thereof, reference is made to the following description, taken in conjunction with the accompanying figures, wherein like reference numerals represent like parts, in which:
FIGURE 1 is a simplified perspective view illustrating an example configuration of an embodiment of a system for filling containers with fluids;
FIGURE 2 is a simplified diagram illustrating a cross-sectional view of example details of an embodiment of the system;
FIGURE 3 is a simplified diagram illustrating other example details of an embodiment of the system;
FIGURE 4 is a simplified diagram illustrating yet other example details of an embodiment of the system;
FIGURE 5 is a simplified diagram illustrating yet other example details of an embodiment of the system;
FIGURE 6 is a simplified diagram illustrating yet other example details of an embodiment of the system;
FIGURE 7 is a simplified diagram illustrating yet other example details of an embodiment of the system; and
FIGURE 8 is a simplified flow diagram illustrating example operations that may be associated with an embodiment of the system.

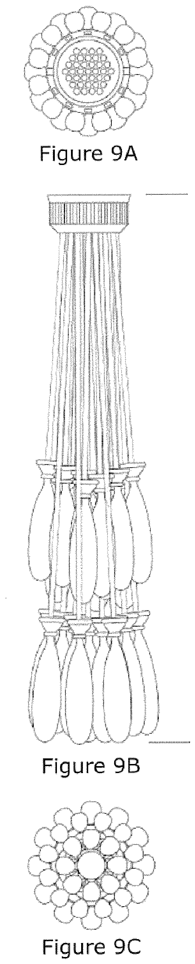

FIGURE 9a is an end view of figure 9b
FIGURE 9b is a side view of an example of the present invention
FIGURE 9c is an opposite end view of figure 9b

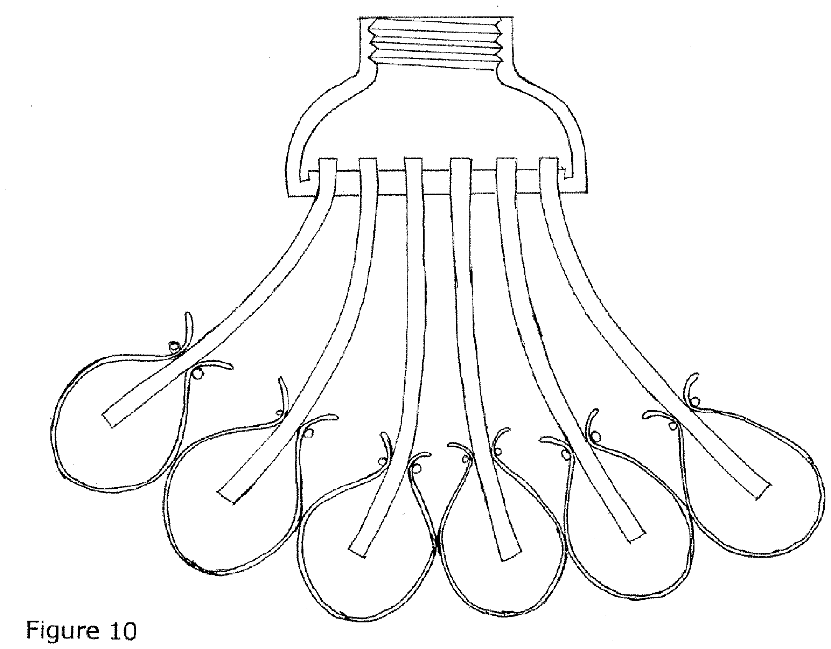

FIGURE 10 is a side view showing the tubes splayed apart more when the balloons have been filled.

### DETAILED DESCRIPTION OF THE INVENTION

In a broad sense the present invention includes or utilizes an apparatus that includes a housing (e.g., casing, manifold, covering, etc. that includes a cavity inside) with a first opening at a first region and a plurality of openings at a second region. , A plurality of hollow tubes extend from the plurality of openings to a plurality of containers (e.g., receptacles, vessels, ampules, test-tubes, balloons, etc.). The tubes may be integrally formed with the housing or separately assembled therewith. The containers are removably attached to the hollow tubes. A plurality of elastic fasteners are utilized. Each elastic fastener clamps a container to a corresponding hollow tube, such that when the containers are filled with fluid and detached from the corresponding hollow tubes, each elastic fastener seals each container to help retain the fluid inside the container. The fasteners may be integrally formed with the container or separately associated therewith.

Some examples of the present invention will now be described with reference to the accompanying drawings. It is to be understood that the following disclosure describes several example embodiments for implementing different features, structures, or functions of the system. Example embodiments of components, arrangements, and configurations are described herein to simplify the present disclosure. However, these example embodiments are provided merely as examples and are not intended to limit the scope of the invention.

The present disclosure may repeat reference numerals and/or letters in the various exemplary embodiments and across the Figures provided herein. This repetitions is for the purpose of simplicity and clarity and does not in itself indicate a relationship between the various exemplary embodiments and/or configurations discussed in the various Figures.

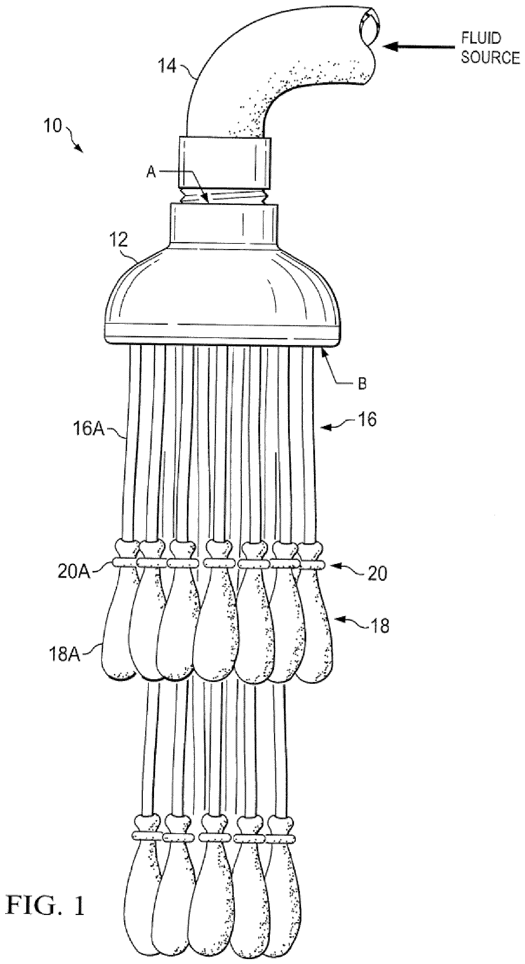

FIGURE 1 is a simplified diagram illustrating an example embodiment of a system 10 for filling containers with fluids. System 10 includes a housing 12 removably attachable to a hose 14 (e.g., tube, pipe, etc.) at a first region such as on a first end A and to a plurality of hollow tubes 16 at a second region such as the second end B.

As used herein, the term "housing" encompasses a hollow space or chamber enclosed by a rigid or semi-rigid casing (e.g., covering, skin, sleeve, sheath, etc.). A manifold arrangement is provide by the housing. In some embodiments, end A may include a threaded opening configured to mate with corresponding threads on hose 14. In some embodiments, end A may be smaller in circumference or area than end B. Hose 14 may be connected to a fluid source, such as a water tank, gas tank, water supply line, etc. on end A. End B may include a plurality of openings such as holes (preferably configured in an array), configured to fit tubes 16. In some embodiments, tubes 16 may be integrally formed with the housing or are permanently attached (e.g., welded, brazed, stuck with adhesives, press-fitted, etc.) to housing 12. In other embodiments, tubes 16 may be removably attached (e.g., with threads, pressure, etc.) to housing 12.

The tubes each have an outlet opening distal the housing. A plurality of containers 18 may be held (e.g., attached, fastened, camped, clinched, secured, etc.) to plurality of hollow tubes 16 at the distal ends of the tube. This is achieved using elastic valves 20 of the type elastic ring. Each tube has one container associated with it.

As used herein, the term "container" refers to an object that can hold something, such as fluids. It is preferably an elastic container but does not need to be so. The term "valve" refers to an object that regulates, directs, or controls the flow of fluids, by opening, closing, or partially obstructing passageways of fluid flow. Elastic valves 20 comprise elastic fasteners, such as O-rings or rubber bands. In an example embodiment, elastic valves 20 comprise corrugations, smocking, elastic fibers, etc. fabricated into the necks of containers 18. They are adapted and configured so that force is required to pull open the necks of containers 18, and removal of the force causes the necks to constrict and close. In yet another example embodiment, elastic valves comprise internal or external plugs affixed to the necks of containers 18, through which tubes 16 may be pushed through to clamp containers 18 thereto.

Note that each of container 18 has an opening to facilitate clamping to tubes 16 and a cavity for containing fluid. For example, one end of an example tube 16A may be fitted through a hole in end B of housing 12, and the other end of tube 16A may be inserted into an example container 18A. An example elastic valve 20A (e.g., O-ring, comprising a mechanical gasket typically in a toroid shape; rubber-band) of sufficient size to expand and clamp around tube 16A may be dispose around (e.g., placed over) a neck (e.g., portion just below opening) of container 18A, clamping and sealing container 18A to tube 16A. Thus, elastic valve 20A may be in an open configuration when container 18A is attached to tube 16A; in elastic valve 20A's open configuration, the neck of container 18A is open, allowing container 18A to fill with fluid. After container 18A is filled with fluid, it may be removed from tube 16A, whereupon elastic valve 20A is free to close and closes, thereby closing the neck of container 18A and sealing the fluid inside.

In one example embodiment, containers 18 may comprise inflatable balloons that may be filled with fluids such as water, air or helium. In another example embodiment, containers 18 may comprise flexible (e.g., stretchy, springy, etc.) elastic containers that may be filled with gaseous or liquid medications. As used herein, the term "elastic" is meant to refer to a property of a material that allows the material to resume its normal shape spontaneously after contraction, dilation, or distortion. In an example, an elastic material may be stretched to 200% of its original length, and the material may return to its original length when the stretching force is removed.

It will be appreciated that the valve may not make a complete seal of the container. An gas or liquid tight seal is preferred but some, minimal, leakage of fluid from the container may occur after removal from the tubes. Any leakage is preferably minimal for the applications to which the container is subsequently put. Eg if its for decorative balloons, slow leakage over a matter of days is likely to be of no consequence. Leakage may also be temporary in that subsequent additional sealing by other steps may take place. Eg decorative balloons may be clipped to a stick that has some sealing functionality to offer additional sealing for the balloon.

In yet another example embodiment, containers 18 may comprise flexible containers that may be filled with body fluids (e.g., urine, blood) for example, to collect multiple samples simultaneously for testing. Virtually any type and kind of fluid may be used within the broad scope of the embodiments. Note that in some embodiments, containers 18 need not be inflatable or flexible in their entireties. For example, a bottom portion of containers 18 may be inelastic (e.g., glass, plastic, metal, etc., of fixed shape and size), and a top portion may be flexible enough to be inserted around tubes 16 and clamped thereon.

When the fluid source is turned on, fluid may flow through housing 12, tubes 16 and fill containers 18. In some embodiments, when housing 12 is connected to a stream of liquid, containers 18 may be filled with the liquid. In some embodiments, the fluid may be supplied at high pressure. Virtually any mechanism that facilitates fluid flow through tubes 16 at sufficient pressure to fill containers 18 may be used within the broad scope of the embodiments. After containers 18 have reached a desired size or volume, they may be detached from tubes 16. In one example embodiment, filled containers 18 may be detached by pulling them away from tubes 16.

In another example embodiment, the connecting force holding filled containers 18 to tubes 16 may be overcome by an upward acceleration on tubes 16, for example, when they are shaken. Thus, filled containers 18 may be detached by shaking housing 12 (or tubes 16) sufficiently vigorously to cause containers 18 to fall off from tubes 16. In some embodiments, the connecting force holding filled container to its corresponding tube is not less than the weight of the filled container; in a specific embodiment, the connecting force holding each container to its corresponding tube is exactly equal to the weight of the filled container. The connecting force may be provided by a combination of constricting forces and friction forces from elastic valves 20.

In yet other embodiments, containers 18 may fall off under gravity; for example, when filled containers 18 reach a threshold weight, they slip off tubes 16 due to gravity. The threshold weight may be based upon the tightness of elastic valves 20, friction between tubes 16 and containers 18, and force from the weight of containers 18 (among other parameters). In various embodiments, containers 14 may slide off tubes 16 and elastic valves 20 may constrict the necks of containers 18, sealing them. In some embodiments, containers 18 may be marked with volumetric measurements, and fluid flow may be turned off when the fluid has filled containers 18 to a desired volume.

The end of the hollow tubes may include a flared outer lip or gradual taper increasing towards the end, that may present a hurdle for the elastic valve when sliding off the tube. An increased force may be applied to the container to overcome this hurdle prior to release from the tube. This may be desirable in order to prevent accidental or premature removal. However selection of an appropriate materials and their co-efficient of friction for the tubes and containers and an appropriately elastic valve, accidental or premature release may also be avoided.

In some embodiments, hollow tubes 16 may be made of a rigid material (e.g., steel, glass); in other embodiments, tubes 16 may be made of a flexible material (e.g., thin plastic). In some embodiments, tubes 16 may be thick, short and rigid; in other embodiments, tubes 16 may be slender, long and flexible. Thus, hollow tubes 16 may be flexible, semi-rigid, or rigid, based on its material of construction, design, or a combination thereof. Note that tubes 16 may be of different lengths, for example, to prevent crowding and to accommodate a larger number of containers 18 than would be possible if tubes 16 were of the same length. Thus, at least some of hollow tubes 16 may be of different lengths than the others.

Also, tubes 16 may be flexible to enable containers 18 to expand. Thus, as containers 18 fill with fluid and expand, they may push against each other, flexing tubes 16. The outermost tubes 16 may be flexed more than the innermost tubes 16 (outer and inner being in reference to a center-point of housing 12, with the inner tubes 16 being closer to the center-point, and the outer tubes 16 being farther from the center-point).

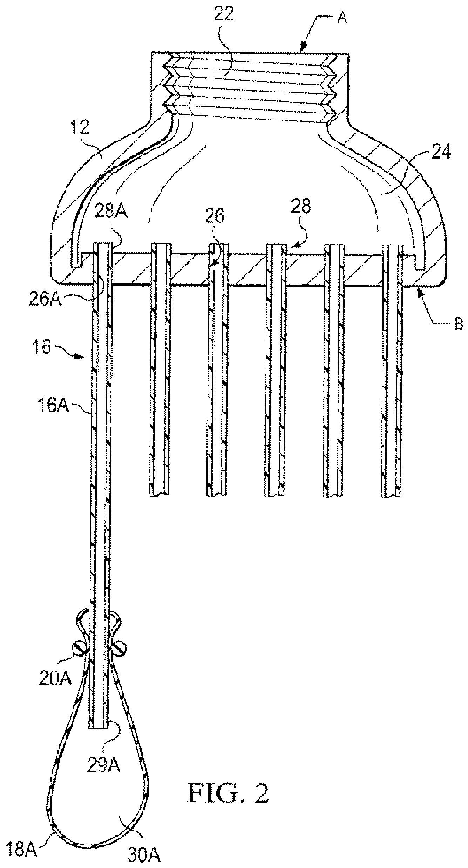

Turning to FIGURE 2, FIGURE 2 is a simplified cross-sectional view of a portion of an embodiment of system 10. Housing 12 comprises a threaded opening 22 at end A, an internal cavity 24, and an array of holes 26 at end B. Internal cavity 24 facilitates distributing the fluid entering at threaded opening 22 to array of holes 26 at end B. In some embodiments, threaded opening 22 may be configured for attaching to a fluid supply hose 14 (e.g., garden hose, plastic tube, etc.). In other embodiments, threaded opening 22 may be attached to corresponding threads in a valve. Array of holes 26 may be configured for connecting first ends 28 of tubes 16 by any suitable means. In some embodiments, first ends 28 of tubes 16 may be connected to corresponding holes 26 by compressing or gluing. In some embodiments, a number of holes 26 in housing 12 and a number of tubes 16 can correspond to a number of containers 18 that are desired to be filled and sealed substantially simultaneously.

To clarify further, only one example tube 16A is shown in the figure. A first end 28A of tube 16A is fitted through a corresponding hole 26A in housing 12. A second end 29A of tube 16A is inserted into container 18A. Elastic valve 20A may be placed around the neck of container 18A clamping the neck to tube 16A. An internal volume 30A of container 18A may be filled with fluid appropriately.

To fill and seal containers 18, housing 12 may be attached to a fluid supply tube (e.g., garden hose) and the fluid supply may be turned on. The fluid enters housing 12, is distributed to holes 26, travels down tubes 16, and fills containers 18. Containers 18 may be filled and may expand substantially simultaneously. When containers 18 have reached a desired size and/or they are filled with the desired volume of fluid, they may be removed from tubes 16. They can be removed by falling off, by shaking them off, by pulling them off by hand, etc. As each container 18A is removed from corresponding tube 16A, respective elastic valve 20A may constrict and close the neck of container 18A, sealing it with the fluid inside.

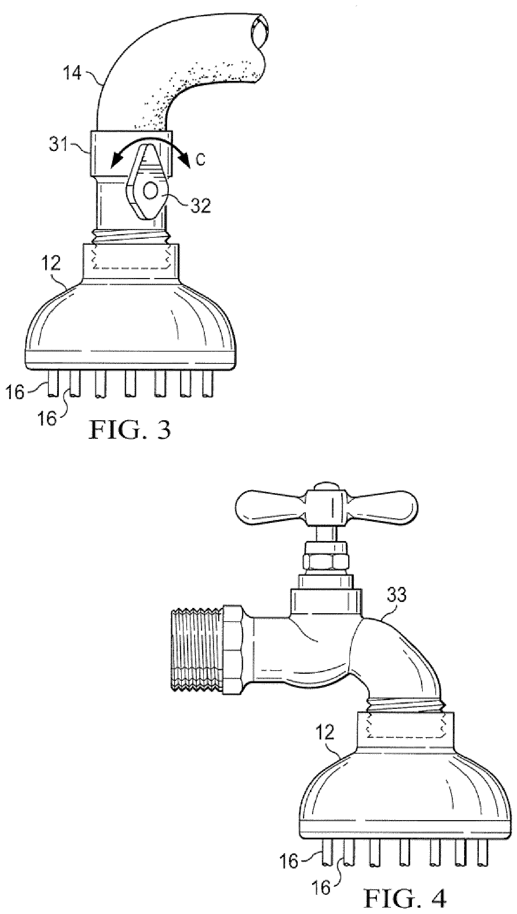

Turning to FIGURE 3, FIGURE 3 is a simplified diagram illustrating example details of a valve 31 that may be attached between hose 14 and housing 12 according to an embodiment of system 10. One end of valve 31 may be attached to hose 14 and the other end may be attached to threaded opening 22 of housing 12 (e.g., using threads). A lever 32 may be turned from one side (of valve 31) to another (e.g., as indicated by arrow C) to turn on and turn off fluid flow to housing 12. For example, to turn on the fluid flow, lever 32 may be turned to a first position; lever 32 may be turned to a second position (e.g., different from the first position) to turn off fluid flow.

Turning to FIGURE 4, FIGURE 4 is a simplified diagram illustrating example details of an embodiment of system 10. Housing 12 may be attached to a spigot 33 (e.g., nozzle, faucet, outlet, etc.) that connects to the fluid source. Spigot 33 may be turned on or turned off to start or stop fluid flow to housing 12.

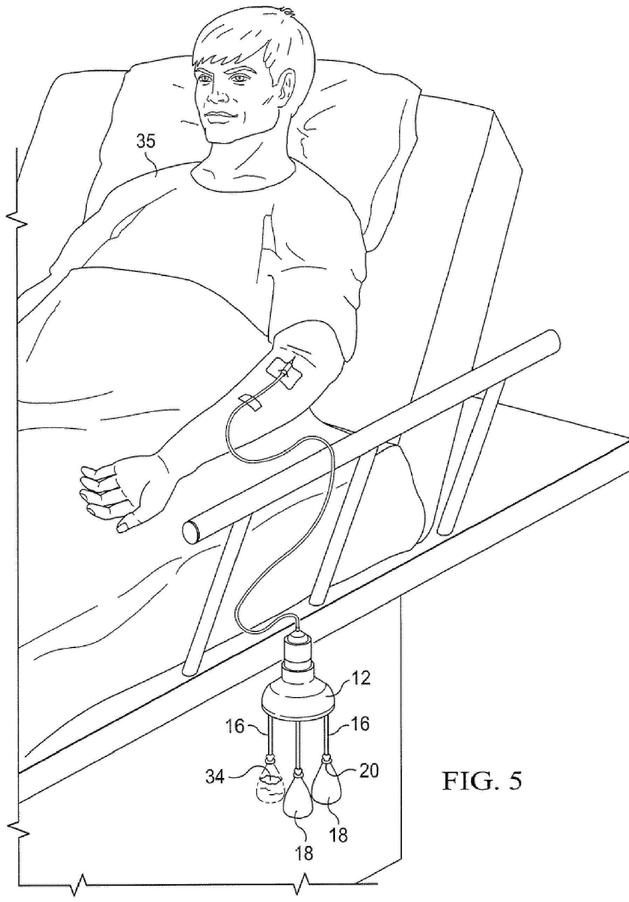

Turning to FIGURE 5, FIGURE 5 is a simplified diagram illustrating example details of an application of an embodiment of system 10. Embodiments of system 10 may be used in a variety of applications, such as for collecting numerous blood samples substantially simultaneously. Blood 34 may be drawn from a human (or animal) and blood 34 may collect substantially simultaneously in plurality of containers 18. The substantial simultaneous collection of blood in such manner can ease patient pain, speed up sampling time, and enable taking multiple samples substantially simultaneously without cross-contamination from one container to another or messy transfers between containers.

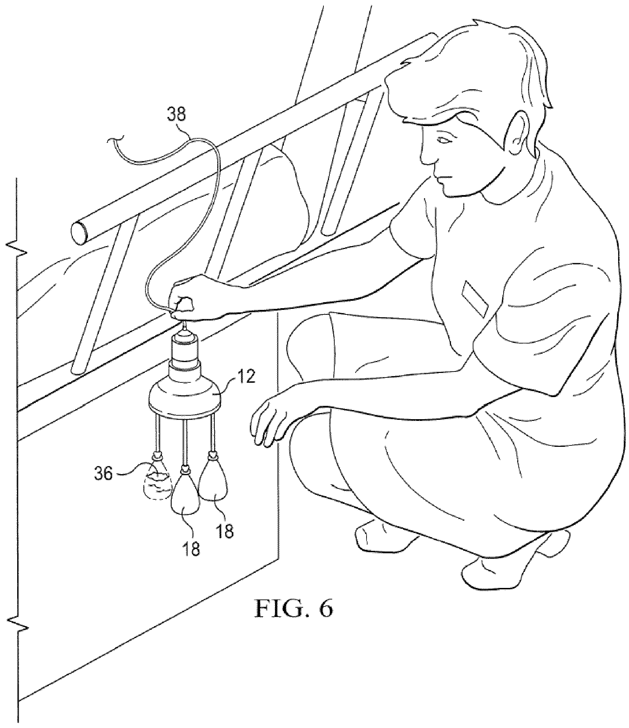

Turning to FIGURE 6, FIGURE 6 is a simplified diagram illustrating example details of an application of an embodiment of system 10. Embodiments of system 10 may be used in a variety of applications, such as for collecting numerous urine samples substantially simultaneously. Urine 36 may be drawn from a human (or animal) through a suitable catheter 38, and may collect substantially simultaneously in plurality of containers 18.

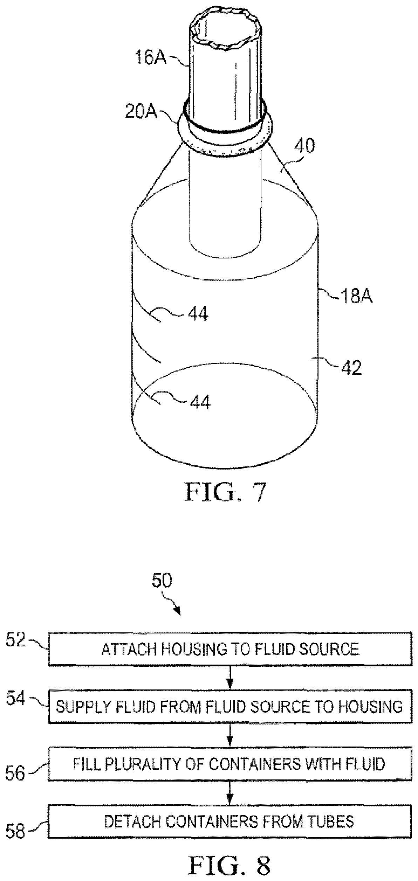

Turning to FIGURE 7, FIGURE 7 is a simplified diagram illustrating example details of an embodiment of system 10. Example container 18A may comprise a flexible portion 40 and an inflexible portion 42. Flexible portion 40 may be clamped on to example tube 16A using example elastic valve 20A. In some embodiments, container 18A may comprise volumetric measurement markings 44. When fluid fills container 18A to a desired volume, for example, as indicated by volumetric measurement marking 44, container 18A may be detached from tube 16A, whereupon elastic valve 20A may close container 18A, sealing the fluid inside.

Turning to FIGURE 8, FIGURE 8 is a simplified flow diagram 50 illustrating example operations that may be associated with an embodiment of system 10. At 52, housing 12 may be attached to a fluid source (e.g., through hose 14, spigot 33, etc.) At 54, fluid may be supplied from the fluid source to housing 12. At 56, plurality of containers 18 may be filled with the fluid. At 58, containers 18 may be detached from corresponding tubes 16.

Note that in this Specification, references to various features (e.g., elements, structures, modules, components, steps, operations, characteristics, etc.) included in "one embodiment", "example embodiment", "an embodiment", "another embodiment", "some embodiments", "various embodiments", "other embodiments", "alternative embodiment", and the like are intended to mean that any such features are included in one or more embodiments of the present disclosure, but may or may not necessarily be combined in the same embodiments.

The elements described herein may be made of any suitable materials, including metal (e.g., stainless steel, copper, brass, bronze, aluminum, etc.), plastic, glass, elastomers, or any suitable combination thereof. Each element may also be made of a combination of different materials (e.g., housing and tubes may be made of plastic and containers may be made of elastic rubber; housing and tubes may be made of stainless steel and containers may be made of a combination of glass and flexible plastic; etc.). Any suitable material or combination of materials may be used for the components described herein without departing from the broad scope of the present disclosure.

In addition, the shapes shown and illustrated in the various FIGURES are for example purposes only. Various other shapes may be used herein without changing the scope of the present disclosure. For example, housing 12 may be conical, cylindrical, pyramidal, etc., without departing from the broad scope of the embodiments. Likewise, tubes 16 may be rigid, or flexible 18 without departing from the scope of the broad embodiments.

## Claims

1. An apparatus for simultaneously filling a plurality of inflatable containers with liquids comprising:
a housing (12) having a first end (A) at which there is an opening configured for connection to a fluid supply, and a plurality of holes from which a plurality of hollow tubes (16) extend,
each of the plurality of hollow tubes (16) having a respective one of the plurality of inflatable containers (18) removably attached at an end thereto to facilitate substantially simultaneous filling of the plurality of containers from the fluid supply, **characterised in that**:
each one of said plurality of inflatable containers is clamped about the end of its respective one of the plurality of hollow tubes by an elastic ring,
the elastic rings are configured to release the inflatable containers from their respective hollow tubes when the inflatable containers have reached a desired size, by one or more of:
i) the inflatable containers falling off the tube;
ii) manual shaking the inflatable containers off the tube by a user; and
iii) the inflatable containers being manually pulled away from the tube by a user,
and each elastic ring is configured to seal its respective one of the plurality of inflatable containers upon detachment of the inflatable container from its corresponding hollow tube.

2. The apparatus of claim 1 wherein the housing has a second end (B) at where the plurality of hollow tubes extend from the plurality of holes, and optionally wherein the second end of the housing is located opposite to the first end of the housing.

3. The apparatus of claim 1 or claim 2 wherein the holes are arranged in an array, and optionally wherein the opening has an effective diameter and the array of holes has an effective diameter, and wherein the effective diameter of the array of holes is greater than the effective diameter of the opening.

4. The apparatus of any one of claims 1 to 3 wherein the opening is threaded and optionally wherein an internal surface of the opening is threaded.

5. The apparatus of any one of claims 1 to 4 wherein each one of the plurality of hollow tubes has been inserted into a respective one of the plurality of holes and compressed or glued into place, or wherein the plurality of hollow tubes are removably attached to said housing.

6. The apparatus of any one of claims 1 to 5 wherein a neck of each one of said plurality of inflatable containers is clamped about the end of its respective one of the plurality of hollow tubes by the elastic ring disposed at the neck of the inflatable container.

7. The apparatus of claim 6 wherein the elastic ring is configured to constrict the neck of the inflatable container in order to seal it upon removal from the hollow tube.

8. The apparatus of any one of claims 1 to 7 wherein said elastic ring is of a lesser diameter than that of the hollow tube, but is of sufficient size to expand and clamp around the tube.

9. The apparatus of any one of claims 1 to 8 wherein the plurality of hollow tubes co-extend from the housing in the same general direction to present the plurality of inflatable containers adjacent to one another in a bunch and optionally wherein the plurality of hollow tubes co-extend from a common face of the housing.

10. The apparatus of any one of claims 1 to 9 wherein the number of holes in the plurality of holes and the number of hollow tubes in the plurality of hollow tubes correspond to the number of inflatable containers in the plurality of inflatable containers which are to be simultaneously filled by the apparatus.

11. The apparatus of any one of claims 1 to 10 wherein the inflatable containers are made of an elastic material.

12. The apparatus of claim 11 wherein the inflatable containers are water balloons.

13. The apparatus of any one of claims 1 to 12 wherein the liquid is water.

14. A method for simultaneously filling a plurality of inflatable containers (18) with liquids, comprising at least the steps of:
A. attaching a housing (12) to a liquid supply via an opening at a first end (A) of the housing,
said housing further comprising a plurality of holes from which a plurality of hollow tubes (16) extend, each of the plurality of hollow tubes having a respective one of the plurality of inflatable containers removably attached at an end thereto to facilitate substantially simultaneous filling of the plurality of containers from the fluid supply,
and wherein each one of said plurality of inflatable containers is clamped about the end of its respective one of the plurality of hollow tubes by an elastic ring configured to restrict detachment of the inflatable container from the hollow tube,
B. simultaneously filling each of said plurality of inflatable containers with liquid,
C. detaching the plurality of inflatable containers from their respective tube ends when the inflatable containers have reached a desired size by one or more of:
i) falling off the tube;
ii) manual shaking by a user; or
iii) being manually pulled away from the tube by a user; and
D. allowing the elastic rings to seal the plurality inflatable containers with the liquid inside after release from the hollow tubes.

15. The method of claim 14 which employs an apparatus for simultaneously filling a plurality of inflatable containers with liquids as claimed in any one of claims 1 to 13.

## Patentansprüche

1. Vorrichtung zum gleichzeitigen Befüllen einer Vielzahl von aufblasbaren Behältern mit Flüssigkeiten, umfassend:
ein Gehäuse (12) mit einem ersten Ende (A), an dem eine Öffnung zur Verbindung mit einer Fluidversorgung konfiguriert ist, und einer Vielzahl von Löchern, aus denen sich eine Vielzahl von Hohlrohren (16) erstrecken, wobei an jedem der Vielzahl von Hohlrohren (16) ein jeweiliger der Vielzahl von aufblasbaren Behältern (18) an einem Ende daran abnehmbar befestigt ist, um ein im Wesentlichen gleichzeitiges Befüllen der Vielzahl von Behältern aus der Fluidversorgung zu ermöglichen, **dadurch gekennzeichnet, dass**:
jeder der Vielzahl von aufblasbaren Behältern durch einen elastischen Ring um das Ende seines jeweiligen aus der Vielzahl von Hohlrohren geklemmt ist,
die elastischen Ringe konfiguriert sind, um die aufblasbaren Behälter aus ihren jeweiligen Hohlrohren zu lösen, wenn die aufblasbaren Behälter eine gewünschte Größe durch Folgendes erreicht haben:
i) Abfallen der aufblasbaren Behälter von dem Rohr;
ii) manuelles Schütteln der aufblasbaren Behälter von dem Rohr durch einen Benutzer; und/oder
iii) manuelles Wegziehen der aufblasbaren Behälter von dem Rohr durch einen Benutzer, wobei jeder elastische Ring konfiguriert ist, um seinen jeweiligen Behälter aus der Vielzahl von aufblasbaren Behältern abzudichten, nachdem der aufblasbare Behälter von seinem entsprechenden Hohlrohr getrennt wird.

2. Vorrichtung nach Anspruch 1, wobei das Gehäuse ein zweites Ende (B) aufweist, an dem sich die Vielzahl von Hohlrohren aus der Vielzahl von Löchern erstrecken, und wobei sich optional das zweite Ende des Gehäuses gegenüber dem ersten Ende des Gehäuses befindet.

3. Vorrichtung nach Anspruch 1 oder Anspruch 2, wobei die Löcher in einer Anordnung angeordnet sind, und wobei optional die Öffnung einen effektiven Durchmesser hat und die Anordnung der Löcher einen effektiven Durchmesser hat, und wobei der effektive Durchmesser der Anordnung von Löchern größer als der effektive Durchmesser der Öffnung ist.

4. Vorrichtung nach einem der Ansprüche 1 bis 3, wobei die Öffnung gewindet ist und wobei optional eine Innenoberfläche der Öffnung gewindet ist.

5. Vorrichtung nach einem der Ansprüche 1 bis 4, wobei jedes der Vielzahl von Hohlrohren in ein jeweiliges Loch der Vielzahl von Löchern eingeführt und auf der Stelle komprimiert oder festgeklebt worden ist, oder wobei die Vielzahl von Hohlrohren an dem Gehäuse abnehmbar befestigt sind.

6. Vorrichtung nach einem der Ansprüche 1 bis 5, wobei ein Hals jedes der Vielzahl von aufblasbaren Behältern um das Ende seines jeweiligen der Vielzahl von Hohlrohren durch den elastischen Ring, der an dem Hals des aufblasbaren Behälters angebracht ist, geklemmt wird.

7. Vorrichtung nach Anspruch 6, wobei der elastische Ring konfiguriert ist, um den Hals des aufblasbaren Behälters zu verengen, um diesen nach der Entfernung von dem Hohlrohr abzudichten.

8. Vorrichtung nach einem der Ansprüche 1 bis 7, wobei der elastische Ring einen kleineren Durchmesser als das Hohlrohr hat, jedoch von ausreichender Größe ist, um zu expandieren und um das Rohr herum zu klemmen.

9. Vorrichtung nach einem der Ansprüche 1 bis 8, wobei sich die Vielzahl von Hohlrohren aus dem Gehäuse gemeinsam in der gleichen allgemeinen Richtung erstrecken, um die Vielzahl von aufblasbaren Behältern angrenzend einander in einer Gruppe zu präsentieren und wobei sich optional die Vielzahl von Hohlrohren von einer gemeinsamen Fläche des Gehäuses aus gemeinsam erstrecken.

10. Vorrichtung nach einem der Ansprüche 1 bis 9, wobei die Anzahl der Löcher in der Vielzahl von Löchern und die Anzahl der Hohlrohre in der Vielzahl von Hohlrohren der Anzahl von aufblasbaren Behältern in der Vielzahl von aufblasbaren Behältern entsprechen, die von der Vorrichtung gleichzeitig zu befüllen sind.

11. Vorrichtung nach einem der Ansprüche 1 bis 10, wobei die aufblasbaren Behälter aus einem elastischen Material hergestellt sind.

12. Vorrichtung nach Anspruch 11, wobei die aufblasbaren Behälter Wasserballons sind.

13. Vorrichtung nach einem der Ansprüche 1 bis 12, wobei die Flüssigkeit Wasser ist.

14. Verfahren zum gleichzeitigen Befüllen einer Vielzahl von aufblasbaren Behältern (18) mit Flüssigkeiten, umfassend wenigstens die folgenden Schritte:
A. Befestigen eines Gehäuses (12) an einer Flüssigkeitsversorgung über eine Öffnung an einem ersten Ende (A) des Gehäuses, wobei das Gehäuse ferner eine Vielzahl von Löchern umfasst, aus denen sich eine Vielzahl von Hohlrohren (16) erstrecken, wobei an jedem der Vielzahl von Hohlrohren an jeweiliger der Vielzahl von aufblasbaren Behältern an einem Ende daran abnehmbar befestigt ist, um ein im Wesentlichen gleichzeitiges Befüllen der Vielzahl von Behältern aus der Fluidversorgung zu ermöglichen, und wobei jeder der Vielzahl von aufblasbaren Behältern durch einen elastischen Ring um das Ende seines jeweiligen aus der Vielzahl von Hohlrohren geklemmt ist, der konfiguriert ist, um das Lösen des aufblasbaren Behälters von dem Hohlrohr einzuschränken,
B. gleichzeitiges Befüllen jedes der Vielzahl von aufblasbaren Behältern mit Flüssigkeit,
C. Ablösen der Vielzahl von aufblasbaren Behältern von ihren jeweiligen Rohrenden, wenn die aufblasbaren Behälter eine gewünschte Größe durch Folgendes erreicht haben:
i. Abfallen von dem Rohr;
ii. manuelles Schütteln durch einen Benutzer; oder
iii. manuelles Wegziehen von dem Rohr durch einen Benutzer; und/oder
D. Ermöglichen, dass die elastischen Ringe die Vielzahl von aufblasbaren Behältern mit der darin enthaltenen Flüssigkeit nach dem Ablösen von den Hohlrohren abdichten.

15. Verfahren nach Anspruch 14, das eine Vorrichtung für das gleichzeitige Befüllen einer Vielzahl von aufblasbaren Behältern mit Flüssigkeiten nach einem der Ansprüche 1 bis 13 anwendet.

## Revendications

1. Appareil de remplissage simultané d'une pluralité de conteneurs gonflables avec des liquides comprenant :
un boîtier (12) ayant une première extrémité (A) au niveau de laquelle se trouve une ouverture conçue pour un raccord à une alimentation en fluide, et une pluralité de trous à partir desquels une pluralité de tubes creux (16) s'étendent, chacun de la pluralité de tubes creux (16) ayant un conteneur respectif de la pluralité de conteneurs gonflables (18) fixé de manière amovible à une extrémité de celui-ci pour faciliter le remplissage sensiblement simultané de la pluralité de conteneurs à partir de l'alimentation en fluide, **caractérisé en ce que** :
chacun de ladite pluralité de conteneurs gonflables est serré autour de l'extrémité de son tube respectif de la pluralité de tubes creux par un anneau élastique,
les anneaux élastiques sont conçus pour libérer les conteneurs gonflables de leurs tubes creux respectifs lorsque les conteneurs gonflables ont atteint une taille souhaitée, d'une ou de plusieurs des façons suivantes :
i) lorsque les conteneurs gonflables tombent du tube ;
ii) lorsque les conteneurs gonflables sont secoués manuellement par un utilisateur jusqu'a sortir du tube ; et
iii) lorsque les conteneurs gonflables sont retirés manuellement du tube par un utilisateur,
et chaque anneau élastique est conçu pour sceller son conteneur respectif de la pluralité de conteneurs gonflables lors du détachement du conteneur gonflable de son tube creux correspondant.

2. Appareil selon la revendication 1, dans lequel le boîtier a une seconde extrémité (B) à l'endroit où la pluralité de tubes creux s'étendent à partir de la pluralité de trous, et facultativement dans lequel la seconde extrémité du boîtier est située à l'opposé de la première extrémité du boîtier.

3. Appareil selon la revendication 1 ou la revendication 2, dans lequel les trous sont disposés en cercle, et facultativement dans lequel l'ouverture présente un diamètre effectif et le cercle de trous présente un diamètre effectif, et dans lequel le diamètre effectif du cercle de trous est supérieur au diamètre effectif de l'ouverture.

4. Appareil selon l'une quelconque des revendications 1 à 3, dans lequel l'ouverture est filetée et facultativement dans lequel une surface interne de l'ouverture est filetée.

5. Appareil selon l'une quelconque des revendications 1 à 4, dans lequel chacun de la pluralité de tubes creux a été inséré dans un trou respectif de la pluralité de trous et comprimé ou collé en place, ou dans lequel la pluralité de tubes creux sont fixés de manière amovible au boîtier.

6. Appareil selon l'une quelconque des revendications 1 à 5, dans lequel un col de chacun de ladite pluralité de conteneurs gonflables est serré autour de l'extrémité de son tube respectif de la pluralité de tubes creux par l'anneau élastique disposé au niveau du col du conteneur gonflable.

7. Appareil selon la revendication 6, dans lequel l'anneau élastique est conçu pour resserrer le col du conteneur gonflable afin de le sceller lors de son retrait du tube creux.

8. Appareil selon l'une quelconque des revendications 1 à 7, dans lequel ledit anneau élastique est d'un diamètre inférieur à celui du tube creux, mais est de taille suffisante pour se dilater et se serrer autour du tube.

9. Appareil selon l'une quelconque des revendications 1 à 8, dans lequel la pluralité de tubes creux s'étendent conjointement à partir du boîtier dans la même direction générale pour présenter la pluralité de conteneurs gonflables adjacents les uns aux autres dans un groupe et facultativement dans lequel la pluralité de tubes creux s'étendent conjointement à partir d'une face commune du boîtier.

10. Appareil selon l'une quelconque des revendications 1 à 9, dans lequel le nombre de trous dans la pluralité de trous et le nombre de tubes creux dans la pluralité de tubes creux correspondent au nombre de conteneurs gonflables dans la pluralité de conteneurs gonflables qui sont à remplir simultanément par l'appareil.

11. Appareil selon l'une quelconque des revendications 1 à 10, dans lequel les conteneurs gonflables sont faits d'un matériau élastique.

12. Appareil selon la revendication 11, dans lequel les conteneurs gonflables sont des ballons d'eau.

13. Appareil selon l'une quelconque des revendications 1 à 12, dans lequel le liquide est de l'eau.

14. Procédé de remplissage simultané d'une pluralité de conteneurs gonflables (18) avec des liquides, comprenant au moins les étapes de :
A. fixation d'un boîtier (12) à une alimentation en liquide par le biais d'une ouverture à une première extrémité (A) du boîtier,
ledit boîtier comprenant en outre une pluralité de trous à partir desquels une pluralité de tubes creux (16) s'étendent, chacun de la pluralité de tubes creux ayant un conteneur respectif de la pluralité de conteneurs gonflables fixés de manière amovible à une extrémité pour faciliter le remplissage sensiblement simultané de la pluralité de conteneurs à partir de l'alimentation en fluide,
et dans lequel chacun de ladite pluralité de conteneurs gonflables est serré autour de l'extrémité de son tube respectif l'un de la pluralité de tubes creux par un anneau élastique conçu pour restreindre le détachement du conteneur gonflable du tube creux,
B. remplissage simultané de chacun de ladite pluralité de conteneurs gonflables avec du liquide,
C. détachement de la pluralité de conteneurs gonflables de leurs extrémités de tube respectives lorsque les conteneurs gonflables ont atteint une taille souhaitée, d'une ou de plusieurs des façons suivantes :
i. lorsqu'ils tombent du tube ;
ii. lorsqu'ils sont secoués manuellement par un utilisateur ; ou
iii. lorsqu'ils sont retirés manuellement du tube par un utilisateur ; et
D. le fait de permettre aux anneaux élastiques de sceller la pluralité de conteneurs gonflables avec le liquide à l'intérieur après libération des tubes creux.

15. Procédé selon la revendication 14, qui utilise un appareil de remplissage simultané d'une pluralité de récipients gonflables avec des liquides selon l'une quelconque des revendications 1 à 13.
